# EUROPEAN PATENT APPLICATION

(11) **EP 4 744 689 A1**
(43) Date of publication of application: **20.05.2026**
(21) Application number: 23959802.2
(22) Date of filing: 29.11.2023
(51) Int. Cl.: A61M 1/06

(54) **PHYSIOTHERAPY STRUCTURE, BREAST PUMP, AND BREAST PUMPING BRASSIERE**

(71) Applicant: Shenzhen Lute Jiacheng Supply Chain Management Co., Ltd., Shenzhen, Guangdong 518000 (CN)
(72) Inventor: CHEN, Wanyuan, Shenzhen, Guangdong 518000 (CN); WEN, Jinsen, Shenzhen, Guangdong 518000 (CN); HU, Yangyang, Shenzhen, Guangdong 518000 (CN)
(74) Representative: Osterhoff, Utz
(86) International application number: PCT/CN2023/135190
(87) International publication number: WO 2025/111883

(57) **Abstract**

The present application discloses a physiotherapy structure, a breast pump and a breast pumping brassiere. The physiotherapy structure includes a breast pump shield, a physiotherapy unit and an electronic control system; the breast pump shield is provided with a first side contacting a breast and a second side not contacting the breast; the physiotherapy unit is abutted against or detachably connected to the second side of the breast pump shield; and the electronic control system is electrically connected to the physiotherapy unit. The technical solutions of the present application aim to facilitate the disassembly of the breast pump shield and the physiotherapy unit in the physiotherapy structure, and reduce damage to the physiotherapy structure.

## Description

### TECHNICAL FIELD

The present application relates to the technical field of maternal and infant products, and in particular to a physiotherapy structure, a breast pump and a breast pumping brassiere.

### BACKGROUND

The breast pump is a tool configured to squeeze out the milk accumulated in the mammary gland. It is generally used when the infant cannot suck milk directly, or when the mother leaves the infant but still wants breast feeding.

The breast pumps in related art include the manual breast pumps and the electric breast pumps. The electric breast pumps include the integrated breast pumps and the split breast pumps. The integrated breast pump usually includes an integrated breast pump shield, a host and a milk storage container. The split breast pump usually integrates the milk storage container and the breast pump shield, and performs the suction control through an external host. However, the breast pump in related art still has technical problems such as single function and inconvenient use, which needs to be solved.

### SUMMARY

The main purpose of the present application is to provide a physiotherapy structure, applied to a breast pump, aiming at facilitating the disassembly of the breast pump shield and the physiotherapy unit in the physiotherapy structure and reducing the damage to the physiotherapy structure.

In order to achieve the above purpose, the physiotherapy structure provided in the present application is applied to the breast pump, including: a breast pump shield, a physiotherapy unit and an electronic control system;
the breast pump shield is provided with a first side contacting a breast and a second side not contacting the breast;
the physiotherapy unit is abutted against or detachably connected to the second side of the breast pump shield; and
the electronic control system is electrically connected to the physiotherapy unit.

In an embodiment, the breast pump shield includes a nipple accommodating portion and a flange, two ends of the nipple accommodating portion are provided with openings and are penetrated, the flange is connected to an opening of the nipple accommodating portion, and the flange is configured to abut against the breast.

In an embodiment, the flange is made of a flexible material or at least partially made of the flexible material.

In an embodiment, the physiotherapy unit includes at least one of a vibration member, a kneading member, a cold compress member or a warm compress member.

The present application also provides a breast pump capable of applying a physiotherapy action to a breast, including: the physiotherapy structure, a host including a housing, a negative pressure assembly, and a milk storage assembly;
the housing is connected to the second side of the breast pump shield or at least partially connected to the breast pump shield, the physiotherapy unit is provided on a surface of the housing towards the breast pump shield, and the physiotherapy unit is configured to apply the physiotherapy action to the breast through the breast pump shield;
the negative pressure assembly is at least partially provided in the housing;
the milk storage assembly is communicated with the breast pump shield; and
the electronic control system is electrically connected to the negative pressure assembly.

In an embodiment, the physiotherapy unit includes a vibration motor configured to vibratingly massage the breast.

In an embodiment, a plurality of vibration motors are provided, the plurality of the vibration motors are provided at intervals along a circumferential direction of the flange or the plurality of the vibration motors are scatterly distributed with the flange as a center.

In an embodiment, the vibration motor is protruded from one surface of the host towards the breast pump shield, one surface of the flange towards the host is provided with a recessed groove, a recessed direction of the recessed groove is away from the host, and the vibration motor is at least partially accommodated in the recessed groove; and
at least a bottom of the recessed groove and/or at least a sidewall of the recessed groove are made of the flexible material, the vibration motor is abutted against the bottom of the recessed groove, the sidewall of the recessed groove is sleeved on at least a part of a structure of the vibration motor, and one side surface of the flange near the breast is flat.

In an embodiment, the flange includes a flange main body, and the recessed groove is integrated or detachably fixed on the flange main body.

In an embodiment, the flange main body is made of a rigid material, and at least part of the recessed groove is made of the flexible material and integrated on the flange main body; or
the flange main body and the recessed groove are integrated, and are made of the flexible material or a semi-flexible material.

In an embodiment, the breast pump further includes a power supply system electrically connected to the negative pressure assembly and the physiotherapy structure and configured to provide a power to the negative pressure assembly and the physiotherapy structure.

The present application also provides a breast pumping brassiere, including: a bra main body and the breast pump;
the bra main body includes a shoulder strap and a shield body; and
the breast pump is provided in the shield body, and the breast pump shield of the breast pump is fitted with the breast.

The present application also provides a breast pump shield, applied in a breast pump device or a breast massage device to be fitted with the breast, including: a flange, a nipple accommodating portion and an elastic deformation structure;
the flange is configured to fit with the breast, the nipple accommodating portion is configured for accommodating nipples, the flange is communicated with the nipple accommodating portion, and the elastic deformation structure is integrated with or detachably connected with the breast pump shield.

In an embodiment, the elastic deformation structure is provided at a connection between the flange and the nipple accommodating portion.

In an embodiment, the elastic deformation structure is provided below a midline of the nipple accommodating portion.

In an embodiment, a plurality of elastic deformation structures are provided, and the plurality of the elastic deformation structures are evenly distributed along a circumferential direction of the flange.

In an embodiment, the flange is made of the flexible material, and a thickness of the flange is greater than a thickness of the elastic deformation structure.

In an embodiment, the nipple accommodating portion is made of the rigid material.

In an embodiment, the breast pump shield further includes: a diaphragm bracket;
the diaphragm bracket is provided on the breast pump shield and is communicated with the breast pump shield, and a pressure diaphragm is provided on the diaphragm bracket.

The present application also provides a breast pump system, including: the breast pump shield configured for fitting with the breast, a negative pressure assembly and a milk storage assembly;
the negative pressure assembly is configured to apply a negative pressure to the breast pump shield to make the elastic deformation structure bulge inward; and
the milk storage assembly is communicated with the breast pump shield so that the milk flows into the milk storage assembly.

The present application also provides a breast pump, including: a host provided with an accommodating chamber and the breast pump system;
the flange of the breast pump shield is connected to one surface of the host, the milk storage assembly is connected to the other surface of the host away from the flange, and the negative pressure assembly is at least partially provided in the accommodating chamber in the host.

The present application also provides a breast pumping brassiere, including: a bra main body and the breast pump;
the bra main body includes a shoulder strap and a shield body; and
the breast pump is provided on the shield body, and the breast pump shield of the breast pump is fitted with the breast.

In an embodiment, a fixing portion is provided on the breast pump, and the fixing portion is detachably connected to the bra main body to fix the breast pump to the bra main body.

In an embodiment, the fixing portion is a clip provided at the host of the breast pump.

The present application also provides a breast pump, including: a breast pump shield, a host and a milk storage assembly;
the breast pump shield includes a flange and a nipple accommodating portion, the flange is configured for fitting with a breast, two ends of the nipple accommodating portion are provided with openings and penetrated for accommodating nipples, and the flange is connected to one end of the nipple accommodating portion;
an accommodating chamber is provided in the housing, a through avoiding hole is provided at a middle of the housing, the other opening of the nipple accommodating portion is penetrated through the avoiding hole, a negative pressure assembly is at least partially provided in the accommodating chamber, and the negative pressure assembly is configured to directly or indirectly apply a negative pressure to the nipple accommodating portion; and
the milk storage assembly is detachably connected to the host and communicated with the other opening of the nipple accommodating portion.

In an embodiment, the breast pump further includes a pressure diaphragm provided between the housing and the breast pump shield;
the negative pressure assembly is configured to apply the negative pressure to the breast pump shield through the pressure diaphragm.

In an embodiment, the pressure diaphragm is detachably sealed or self-sealed and is provided on the breast pump shield, so that in response to that the negative pressure assembly moves, the pressure diaphragm is configured to deform to generate the negative pressure in the breast pump shield.

In an embodiment, a diaphragm bracket is provided on one side of the breast pump shield towards the host, the pressure diaphragm is provided on the diaphragm bracket, and the diaphragm bracket is sealingly communicated with the negative pressure assembly.

In an embodiment, the diaphragm bracket includes a supporting frame, a cover and a connecting structure, and the supporting frame is a bowl-shaped structure with openings at both ends; an opening of the supporting frame is communicated with the breast pump shield, the cover is configured to cover the other opening of the supporting frame, and the pressure diaphragm is sandwiched between the cover and the other opening of the supporting frame; one end of the connecting structure is connected and communicated with the cover, and the other end of the connecting structure is communicated with the negative pressure assembly; and

a rotary connecting member and a clamping member are provided on a peripheral side of the cover, the rotary connecting member is rotationally connected to an edge of the other opening of the supporting frame, and the clamping member is snap-connected to the edge of the other opening of the supporting frame.

In an embodiment, the pressure diaphragm is sealingly connected to the housing.

In an embodiment, a gravity center of the host is above an axis of the nipple accommodating portion.

In an embodiment, one surface of the housing of the host towards the milk storage assembly is a first inclined surface, one surface of the milk storage assembly towards the host is a second inclined surface, and the first inclined surface is fitted with the second inclined surface.

In an embodiment, the milk storage assembly includes a milk bowl cover and a bowl body, the bowl body is provided with a milk storing space with an opening, the milk bowl cover is configured to cover the opening, the milk bowl cover is provided with a through hole, and the other end of the nipple accommodating portion is communicated with the bowl body through the through hole, so that the milk flows into the bowl body.

In an embodiment, an assembly cylinder is provided on the milk bowl cover; one end of the assembly cylinder is connected to an edge of the through hole, the assembly cylinder is provided in the milk storing space, and the other end of the assembly cylinder is provided with a milk flowing hole; the other end of the nipple accommodating portion is penetrated through the assembly cylinder;
a first sealing member is provided between the nipple accommodating portion and the assembly cylinder;
a sidewall of the assembly cylinder is configured to extend inward in a circumferential direction to form a protruding flange, the other end of the nipple accommodating portion is provided with the first sealing member, and the nipple accommodating portion is configured to presse the first sealing member against the protruding flange to seal; and
the first sealing member is a suction cup.

In an embodiment, the milk bowl cover is detachably connected to the bowl body;
a second sealing member is provided between the milk bowl cover and the bowl body;
the second sealing member is a flexible or semi-flexible sealing ring;
the second sealing member is integrated in one of the milk bowl cover and the bowl body, and is sealingly connected to the other one of the milk bowl cover and the bowl body; or
the second sealing member is detachably connected between the milk bowl cover and the bowl body.

In an embodiment, the milk storage assembly further includes a check valve provided at the other end of the assembly cylinder and is communicated with the milk flowing hole to prevent the milk in the bowl body from flowing back;
the check valve includes a connecting cylinder, one end of the connecting cylinder is connected to the assembly cylinder, and the other end of the connecting cylinder is configured to extend downward to form a duckbill valve end; and
one side of the connecting cylinder away from the duckbill valve end is configured to extend outward to form a buckle portion.

In an embodiment, a milk pouring hole is provided near a top of the bowl body, and the milk pouring hole is communicated with the milk storing space; and
one side of the milk bowl cover towards the bowl body is provided with a baffle, and the baffle is provided relative to the milk pouring hole.

In an embodiment, a detachable structure is provided between the host and the milk storage assembly so that the host is detachably connected to the milk storage assembly;
the detachable structure includes an unlock key and a lock structure, the lock structure has a locked state that is snap-connected to the host and the milk storage assembly and an unlocked state that is separated from the host and the milk storage assembly, and the unlock key is connected to the lock structure and is configured to control the lock structure to switch from the locked state to the unlocked state; and
the unlock key is one of a pressing key, a pulling key, a sliding key and a rotating key.

In an embodiment, the detachable structure includes a first magnetic portion provided on the host and a second magnetic portion provided on the milk storage assembly, and the first magnetic portion is magnetically connected to the second magnetic portion.

In an embodiment, an outer edge of the breast pump shield is configured to extend outward and is folded to form a folding portion, and the folding portion is configured to cover at least part of the housing of the host.

In an embodiment, the host includes a charging hole covered by the folding portion.

In an embodiment, an entire peripheral wall of the housing is wrapped by the folding portion.

The present application also provides a breast pump system, including: a breast pump shield, a milk storage assembly, a host including a housing, a power supply system, and a remote control;
the breast pump shield is configured for fitting with the breast;
the milk storage assembly is communicated with the breast pump shield and configured to receive and store a milk;
an accommodating chamber is provided in the housing, and the accommodating chamber is provided with a negative pressure assembly configured to apply a negative pressure to the milk storage assembly or the breast pump shield;
the power supply system is provided in the accommodating chamber and electrically connected to the negative pressure assembly; and
the remote control is configured to communicate with the negative pressure assembly and remotely control the negative pressure assembly to operate and stop; wherein the remote control includes a housing and a control circuit board, the housing of the remote control is provided with a cavity body, and the control circuit board is provided in the cavity body; the housing of the remote control is provided with a control panel, the control panel is electrically connected to the control circuit board, and the remote control is detachably provided on an outer wall of the housing of the host or an outer wall of the milk storage assembly.

In an embodiment, an accommodating groove is provided at a top of the housing, and the remote control is accommodated in the accommodating groove and is detachably connected to an inner wall of the accommodating groove.

In an embodiment, the breast pump system further includes a third magnetic portion and a fourth magnetic portion magnetically attracted to the third magnetic portion;
the third magnetic portion and the fourth magnetic portion are respectively provided on the housing of the remote control and an outer wall of the housing of the remote control.

In an embodiment, the remote control is detachably provided on the housing of the host and connected to the power supply system to provide power;
a connection mode between the remote control and the power supply system is one of a metal contact connection, a wireless connection or a wired connection;
the remote control further includes a display screen provided with the housing of the remote control and electrically connected to the control circuit board; and
the remote control further includes a watch strap configured to connect to the housing of the remote control.

In an embodiment, the milk storage assembly further includes a dust cover detachably connected to the flange of the breast pump shield, and the remote control is detachably provided on the dust cover; and
a convex plate is formed at a middle of the dust cover, and the remote control is clamped to the convex plate so that the remote control is connected to the dust cover.

In the technical solution of the present application, the first side of the breast pump shield is fitted with the breast; the physiotherapy unit is abutted against or detachably connected to the second side of the breast pump shield which does not contact the breast, and the electronic control system is electrically connected and controls the start or stop of the physiotherapy unit. The physiotherapy unit indirectly massages the breast by fitting with the breast pump shield of the breast, achieving the effect of health care massaging. The physiotherapy unit is abutted against or detachably connected on the second side of the breast pump shield that does not contact the breast, which will not be affected when cleaning the breast pump shield, and does not require a connector with the breast pump shield, reducing the possibility of the damage to the connector from repeated unplugging and plugging, thus improving the service life of the physiotherapy structure.

### BRIEF DESCRIPTION OF THE DRAWINGS

In order to illustrate the technical solutions in the embodiments of the present application or in the related art more clearly, the following briefly introduces the accompanying drawings required for the description of the embodiments or the related art. Obviously, the drawings in the following description are only part of embodiments of the present application. For those skilled in the art, other drawings can also be obtained according to the structures shown in these drawings without any creative effort.
FIG. 1 is an exploded view of a breast pump according to an embodiment of the present application.
FIG. 2 is a schematic structural view of a physiotherapy structure in FIG. 1 according to an embodiment of the present application.
FIG. 3 is a schematic structural view of the breast pump according to an embodiment of the present application.
FIG. 4 is a schematic structural view of a diaphragm bracket in the breast pump according to an embodiment of the present application.
FIG. 5 is a schematic structural view of a milk storage assembly in the breast pump according to an embodiment of the present application.
FIG. 6 is a schematic structural view of a host in the breast pump according to an embodiment of the present application.
FIG. 7 is a schematic structural view of the host in the breast pump according to an embodiment of the present application.
FIG. 8 is a schematic internal structural view of the host in the breast pump according to an embodiment of the present application.
FIG. 9 is a schematic structural view of the host and a remote control in the breast pump according to an embodiment of the present application.
FIG. 10 is a schematic structural view of a dust cover and the remote control in the breast pump according to an embodiment of the present application.
FIG. 11 is a schematic internal structural view of the host in the breast pump according to an embodiment of the present application.
FIG. 12 is a schematic structural view of a detachable structure in FIG. 11 according to an embodiment of the present application.
FIG. 13 is a schematic module view of a physiotherapy structure in the breast pump according to an embodiment of the present application.
FIG. 14 is a cross-sectional view of the breast pump according to an embodiment of the present application.
FIG. 15 is a schematic structural view of a vibration motor in FIG. 14 according to an embodiment of the present application.
FIG. 16 is a schematic structural view of the remote control and a watch strap in the breast pump according to an embodiment of the present application.
FIG. 17 is a schematic structural view of a breast pumping brassiere according to an embodiment of the present application.
FIG. 18 is an exploded view of the breast pump according to an embodiment of the present application.
FIG. 19 is a cross-sectional view of the breast pump according to an embodiment of the present application.

### Description of reference signs:

| Reference sign | name | Reference sign | name |
|---|---|---|---|
| 1000 | breast pumping brassiere | 21111 | connecting cylinder |
| 100 | breast pump | 21112 | duckbill valve end |
| 10 | physiotherapy assembly | 22 | bowl body |
| 11 | breast pump shield | 221 | milk pouring hole |
| 111 | nipple accommodating portion | 222 | baffle |
| 1111 | first sealing member | 23 | second sealing member |
| 112 | flange | 30 | host |
| 1121 | elastic deformation structure | 31 | housing |
| 1122 | flange main body | 311 | front housing |
| 1123 | folding portion | 312 | rear housing |
| 113 | recessed groove | 313 | avoiding hole |
| 114 | diaphragm bracket | 314 | accommodating groove |
| 1141 | pressure diaphragm | 32 | negative pressure assembly |
| 1142 | supporting frame | 33 | detachable structure |
| 1143 | cover | 331 | unlock key |
| 1144 | connecting structure | 332 | lock structure |
| 12 | physiotherapy unit | 34 | charging hole |
| 121 | vibration motor | 40 | remote control |
| 13 | electronic control assembly | 41 | display screen |
| 20 | milk storage assembly | 42 | watch strap |
| 21 | milk bowl cover | 50 | power supply system |
| 211 | assembly cylinder | 60 | dust cover |
| 2111 | check valve | 200 | bra main body |

The realization of the objective, functional characteristics, and advantages of the present application are further described with reference to the accompanying drawings.

### DETAILED DESCRIPTION OF THE EMBODIMENTS

The technical solutions of the embodiments of the present application will be described in more detail below with reference to the accompanying drawings. It is obvious that the embodiments to be described are only some rather than all of the embodiments of the present application. All other embodiments obtained by those skilled in the art based on the embodiments of the present application without creative efforts shall fall within the scope of the present application.

It should be noted that all directional indications (such as up, down, left, right, front, back, etc.) in the embodiments of the present application are only used to explain the relative positional relationship, the movement situation, etc. among various assemblies under a certain posture as shown in the drawings. If the specific posture changes, the directional indication also changes accordingly.

In addition, the descriptions of "first", "second", etc. are only for the purpose of description, and should not be construed as indicating or implying relative importance or implicitly indicates the number of technical features indicated. Thus, a feature delimited with "first", "second" may expressly or implicitly include at least one of that feature. In addition, the technical solutions between the various embodiments can be combined with each other, but must be based on the realization by those skilled in the art. When the combination of technical solutions is contradictory or cannot be realized, it should be considered that the combination of such technical solutions does not exist or fall within the scope of protection claimed in the present application.

The present application provides a physiotherapy structure.

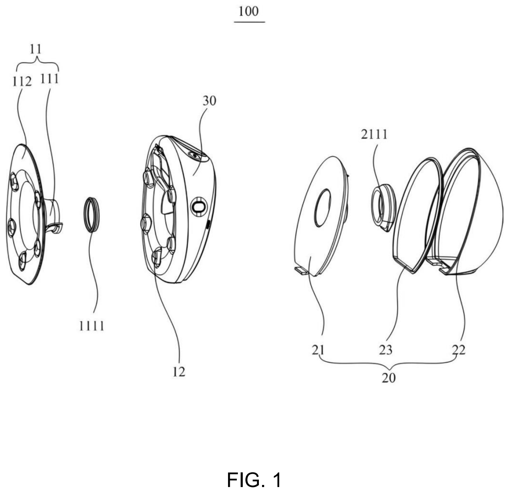

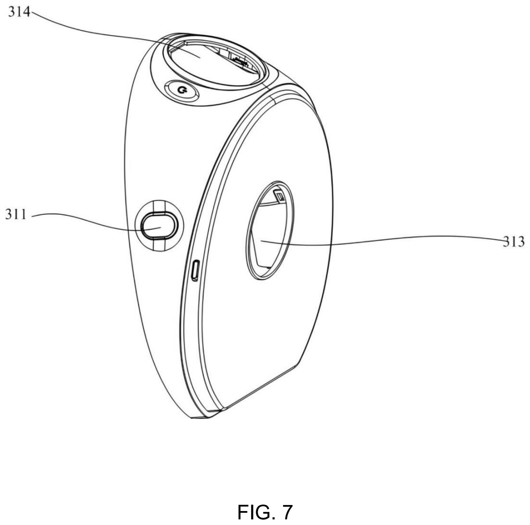

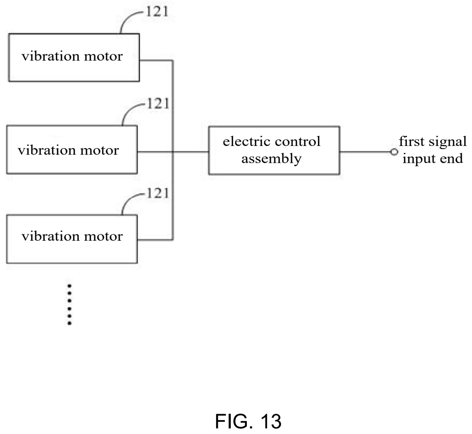

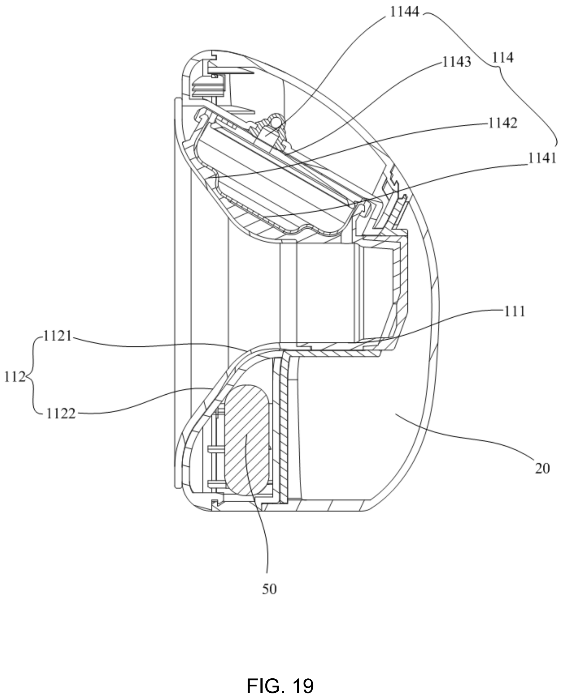

In an embodiment, with reference to FIG. 1 to FIG. 19, the physiotherapy structure, applied to a breast pump 100, includes a breast pump shield 11, a physiotherapy unit 12 and an electronic control system. The breast pump shield 11 has a first side contact with the breast and a second side not contact with the breast, the physiotherapy unit 12 is abutted against or detachably connected to the second side of the breast pump shield 11, and the electronic control system is electrically connected to the physiotherapy unit 12.

In an embodiment, the breast pump shield 11 is configured to fit with the user's breast. A shape of the breast pump shield 11 is a funnel shape or a hemispherical shape that can fit with the user's breast. In order to improve the user's experience, the material of the breast pump shield 11 is made of skin-friendly and soft material, such as silica gel or latex, which are more comfortable and less likely to cause skin allergies and other problems for users. The physiotherapy unit 12 is abutted against or detachably connected to the breast pump shield 11. When the breast pump shield 11 needs to be cleaned after use, it is convenient to separate the physiotherapy unit 12 from the breast pump shield 11. There is no connector between the physiotherapy unit 12 and the breast pump shield 11, which also reduces the possibility of a damage caused by repeated disassembly of the connector, thereby increasing the service life of the physiotherapy structure. The physiotherapy unit 12 is a vibration massage mechanism and is correspondingly provided as a vibration motor 121. It can be understood that, in an embodiment, the physiotherapy unit 12 is a kneading massage mechanism and is correspondingly provided as a kneading massage head. In an embodiment, the physiotherapy unit 12 has a combined function of vibration massage and electrical stimulation, and is correspondingly provided as an electrical stimulation vibration massage head. In an embodiment, the physiotherapy unit 12 has a warm compress or a cold compress effect, and is correspondingly provided as a warm compress piece or a cold compress piece. There are more of the specific implementation of the physiotherapy unit 12, which will not be listed here. The electronic control system is electrically connected to the physiotherapy unit 12 to control a start and stop of the physiotherapy unit 12. The electronic control system and the physiotherapy unit 12 can be controlled through a wired connection, or through a wireless connection such as Bluetooth or WIFI, which is not limited here.

In the technical solution of the present application, the first side of the breast pump shield 11 is fitted with the breast, the physiotherapy unit 12 is abutted against or detachably connected to the second side of the breast pump shield 11 which does not contact the breast, and the electronic control system is electrically connected and controls the start or stop of the physiotherapy unit 12. The physiotherapy unit 12 indirectly massages the breast by fitting with the breast pump shield 11 of the breast, achieving the effect of health care massaging. The physiotherapy unit 12 is abutted against or detachably connected on the second side of the breast pump shield 11 that does not contact the breast, which will not be affected when cleaning the breast pump shield 11, and does not require a connector with the breast pump shield 11, reducing the possibility of damage to the connector from repeated unplugging and plugging, thus improving the service life of the physiotherapy structure.

In an embodiment, with reference to FIG. 1, the breast pump shield 11 includes a nipple accommodating portion 111 and a flange 112. Two ends of the nipple accommodating portion 111 are provided with openings and are penetrated. The flange 112 is connected to an opening of the nipple accommodating portion 111 and is configured for abutting against the breast.

In an embodiment, the breast pump shield 11 includes the flange 112 and the nipple accommodating portion 111. Two ends of the nipple accommodating portion 111 are provided with the openings and penetrated for accommodating the nipples. The material of the nipple accommodating portion 111 can be a rigid material, such as plastic, metal tube, and glass tube, etc., and can also be a flexible material or a semi-flexible material, such as rubber or silica gel, etc., which is not limited here. The flange 112 is in a funnel shape or a hemisphere shape fitted with the breast. The flange 112 is made of the flexible material, such as silica gel, rubber, etc., which is comfortable for users, or it can be made of other materials, such as plastic. The flange 112 is connected to the opening of the nipple accommodating portion 111, which enables the nipple to be in the nipple accommodating portion 111 while the flange 112 is fitted with the breast. The physiotherapy unit 12 performs an indirect massage or kneeing on the breast through the flange 112.

In an embodiment, the flange 112 is made of the flexible material or at least partially made of the flexible material.

In an embodiment, the flange 112 is made of the flexible material that is harmless to the human body and will not cause allergies, such as silica gel or rubber, which can better fit with the user's breast. In an embodiment, at least part of the flange 112 is made of the flexible material, for example, the part contacting the breast is made of the flexible material, which improves the user's comfort.

In an embodiment, as shown in FIG. 1, the physiotherapy unit 12 includes at least one of a vibration member, a kneading member, a cold compress member, or a warm compress member.

In an embodiment, the physiotherapy unit 12 can be the vibration member, the kneading member, the cold compress member, the warm compress member, or various combinations of the above elements. The vibration member can perform a high-frequency vibration massage on the user's breast. Through the vibration massage, the milk discharge effect of the user is better. The kneading member can perform the kneading massage on the user's breast by imitating the kneading movements of the human hand, so that the milk discharge effect of the user is better. The cold compress member and the warm compress member perform the physiotherapy on the user's breast through the cold compress and the warm compress respectively, which can promote the blood circulation to remove blood stasis and relieve swelling and pain.

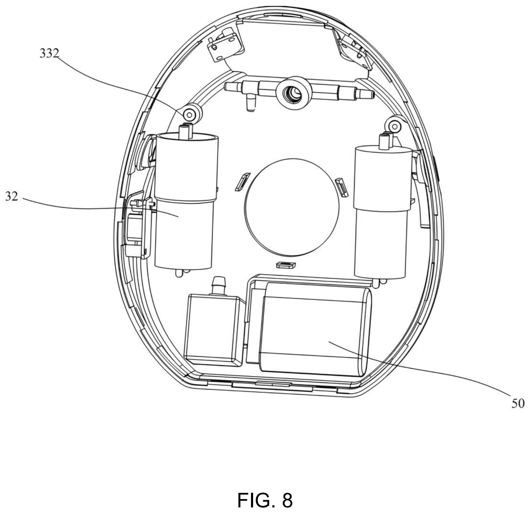

The present application also provides a breast pump 100, with reference to FIG. 1 and FIG. 8, capable of applying the physiotherapy action to the breast. The breast pump 100 includes the physiotherapy structure as above, a host 30, a negative pressure assembly 32 and a milk storage assembly 20. The host 30 includes a housing connected to the second side of the breast pump shield 11 or at least partially connected to the breast pump shield 11, the physiotherapy unit 12 is located on a surface of the housing towards the breast pump shield 11, and the physiotherapy unit 12 applies the physiotherapy action to the breast through the breast pump shield 11. The negative pressure assembly 32 is at least partially located inside the housing, the milk storage assembly 20 is communicated with the breast pump shield 11, and the electronic control system is electrically connected to the negative pressure assembly 32.

In an embodiment, the physiotherapy structure is fitted with the user's breast, and can perform the physiotherapy massage on the user's breast. The host 30 is electrically connected to the electronic control system of the physiotherapy structure, and can power and control the electronic control system, thereby controlling the physiotherapy unit 12 to start or stop. The host 30 is connected to the second side of the breast pump shield 11. The physiotherapy unit 12 is provided on the surface of the host 30 towards the breast pump shield 11. The physiotherapy unit 12 can be directly abutted against the breast pump shield 11 and indirectly perform the physiotherapy massage on the user's breast through the breast pump shield 11. The negative pressure assembly 32 is electrically connected to the host 30, and the host 30 can power or control the negative pressure assembly 32. The milk storage assembly 20 is communicate with the breast pump shield 11, which can be indirectly communicated through the pipeline, or directly communicated, which is not limited here. The milk storage assembly 20 is configured to collect and temporarily store the milk.

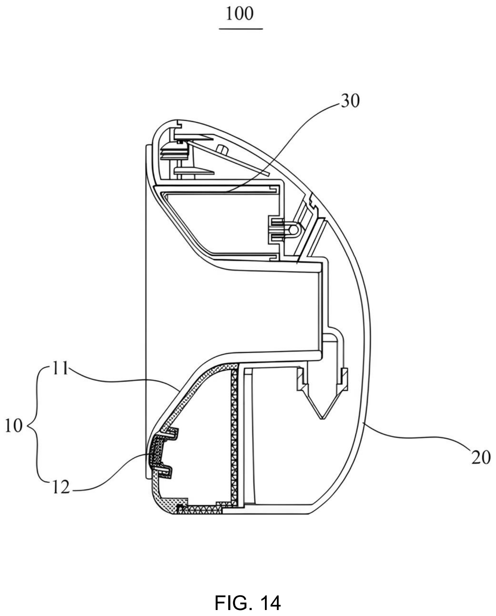

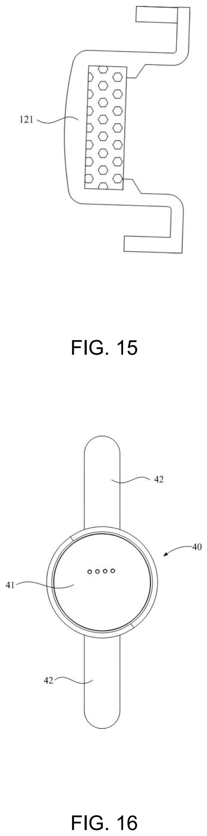

In an embodiment, referring to FIG. 14 and FIG. 15, the physiotherapy unit 12 includes a vibration motor 121 to vibratingly massage the breast.

In an embodiment, the physiotherapy unit 12 includes the vibration motor 121. The vibration motor 121 can vibrate the user's breast through the cooperation of the vibration motor and the vibration head, which promotes the blood circulation to remove blood stasis, unclogs the mammary ducts, and improves the efficiency of milk discharge.

In an embodiment, a plurality of vibration motors 121 are provided at intervals along a circumferential direction of the flange 112 or the plurality of vibration motors 121 are scatterly distributed with the flange 112 as the center.

In an embodiment, a plurality of the vibration motors 121 are provided, which can be configured to vibrate the user's breast at multiple points simultaneously or in rounds, promote blood circulation to remove blood stasis, unclog the mammary ducts, and further improve the efficiency of milk discharge. The plurality of vibration motors 121 are provided at intervals along the circumferential direction of the flange 112 to prevent mutual interference, increase the massage area, and improve the efficiency of milk discharge. In an embodiment, the plurality of vibration motors 121 are scatteredly distributed with the flange 112 as the center, which can vibratingly massage the user's breast at multiple points simultaneously or in rounds, promote blood circulation to remove blood stasis, and unclog the mammary ducts, further improve the efficiency of milk discharge.

In an embodiment, the cross section of the vibration motor 121 is arc-shaped, which can be better fitted with the user's breast and increase the physiotherapy area of a single vibration motor 121, further improving the efficiency of milk discharge. A plurality of vibration motors 121 are provided at intervals along the circumferential direction of flange 112, which can vibrate the user's breast at multiple points simultaneously or in rounds to massage, promote blood circulation to remove blood stasis, and unclog the mammary ducts, further improve the efficiency of milk discharge.

In an embodiment, the cross section of the vibration motor 121 is annular, which can be better fitted with the user's breast, further increasing the physiotherapy area of a single vibration motor 121, thereby improving the efficiency of milk discharge. The circular shape of the annular vibration motor 121 coincides with the center of the flange 112 to vibrate the user's breast evenly. The plurality of the vibration motors 121 can perform multi-ring simultaneous or multi-ring patrol vibration massage effect on the user's breast, which can be better fitted with the user's breast, further increase the physiotherapy area of a single vibration motor 121, and improve the efficiency of milk discharge.

In an embodiment, as shown in FIG. 1 and FIG. 15, the vibration motor 121 protrudes from the surface of the host 30 towards the breast pump shield 11, the surface of the flange 112 towards the host 30 is provided with a recessed groove 113, a recessed direction of the recessed groove 113 is away from the host 30, and the vibration motor 121 is at least partially accommodated in the recessed groove 113. At least the bottom and/or at least the wall of the recessed groove 113 is made of the flexible material, the vibration motor 121 is abutted against the bottom of the recessed groove 113, and the sidewall of the recessed groove 113 is sleeved on at least part of the structure of the vibration motor 121. The side of the flange 112 close to the breast is a flat surface.

In an embodiment, the vibration motor 121 is exposed on the surface of the host 30 towards the breast pump shield 11. Through the recessed groove 113 provided on the flange 112, the vibration motor 121 is partially accommodated in the recessed groove 113 to indirectly vibrate the user's breast, which avoids the damage to the user's skin caused by a rigid impact generated by the vibration motor 121. The bottom and the wall of the recessed groove 113 are made of the flexible material, which can be abutted against the bottom of the recessed groove 113 by the vibration motor 121, so that the sidewall of the recessed groove 113 is sleeved on the outer peripheral wall of the vibration motor 121, and the bottom and the wall of the recessed groove 113 are made of the flexible material, which can effectively avoid the influence on other parts of the flange 112 when the vibration motor 121 moves. The vibration of the vibration motor 121 is transmitted to the breast through the recessed groove 113, which has a higher efficiency and a better massage effect.

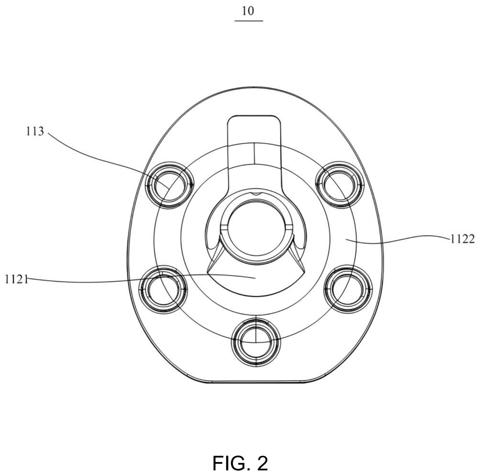

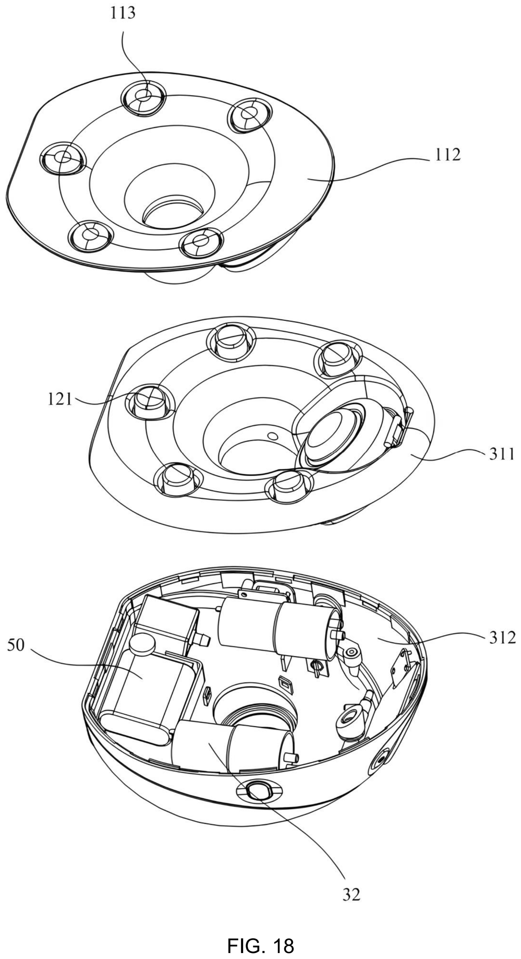

In an embodiment, with reference to FIG. 2 and FIG. 18, the flange 112 includes a flange main body 1122. The recessed groove 113 is integrated or detachably fixed on the flange main body 1122.

In an embodiment, the recessed groove 113 and the flange main body 1122 can be integrated, which is convenient for processing and does not require a secondary assembly, and the vibration motor 121 can be directly sleeved in the recessed groove 113. It can be understood that, the recessed groove 113 and the flange main body 1122 can also be detachably connected, such as a snap connection, etc., so as to facilitate the disassembly of a single recessed groove 113 and the replacement or repair of the corresponding vibration motor 121.

A flexible wrinkle is provided at the connection between the flange main body 1122 and the recessed groove 113. In this way, the wrinkled part of the flange main body 1122 can effectively avoid the influence on the main body of the flange main body 1122 when the vibration motor 121 moves. A surface of the recessed groove 113 towards the vibration motor 121 is provided with a first socket. A notch of the first socket is expanded, and the vibration motor 121 is located in the first socket, which makes it easier to assemble the vibration motor 121 into the first socket.

The vibration motor 121 may include an elastic cap and the vibration motor. The elastic cap is installed in the installation cavity and extends from the through hole. The elastic cap is provided with a second socket, and the vibration motor is embedded in the second socket. In this way, the vibration motor 121 can massage the user through vibration, promote blood circulation to remove blood stasis, unclog the mammary ducts, and improve the efficiency of milk discharge. In addition, when the vibration motor vibrates, the elastic cap can prevent the rigid impact of the vibration motor to the wall of the through hole, so as to avoid the problem of abnormal noise and component wear.

The elastic cap is installed in the installation cavity in various ways. A cap brim is provided on the outer periphery of one end of the elastic cap located in the installation cavity, and the cap brim is bonded to the wall of the installation cavity. A cap band is formed on the outer periphery of one end of the elastic cap close to the installation cavity. An assembly ring is provided on the wall of the installation cavity on the outer periphery of the through hole, and the cap band is sleeved outside the assembly ring. In this way, there is no need to use additional materials and devices to install the elastic cap, and it can be installed with bare hands, making the installation more efficient and convenient.

A limiting rib is provided on the outer periphery of the assembly ring, a limiting breach is correspondingly provided on the cap band, and the limiting rib is cooperated with the limiting breach, so that the limiting rib can limit the cap band, prevent the problem of the guide torsion of the vibrating motor knotting caused by the rotation of the elastic cap, and strengthen the structure.

The circumferential side of the elastic cap is spaced apart from the wall of the through hole. In this way, when the vibration motor vibrates, it can avoid the fatigue deformation caused by repeated extrusion of the peripheral side wall of the elastic cap and the wall of the through hole.

In an embodiment, the flange main body 1122 is made of the rigid material, and the recessed groove 113 is at least partially made of the flexible material and integrated on the flange main body 1122, or the flange main body 1122 and the recessed groove 113 are integrated, and both of which are made of the flexible or semi-flexible material..

In an embodiment, the flange main body 1122 is made of the rigid material, such as plastic, etc., and the recessed groove 113 is at least partially made of the flexible material, such as silica gel or rubber, etc., and the vibration effect of the vibration motor 121 is transmitted to the breast through the recessed groove 113, which is at least partially made of the flexible material, to improve the vibration efficiency. In an embodiment, the flange main body 1122 and the recessed groove 113 are both made of the flexible or semi-flexible materials, such as rubber or silica gel, etc., and the flange main body 1122 and the recessed groove 113 are integrated to facilitate production and processing. The vibration motor 121 transmits vibration effect to the breast through the recessed groove 113 and the flange main body 1122, which can improve the vibration efficiency.

In an embodiment, the physiotherapy unit 12 may also include a massage assembly to promote blood circulation to remove blood stasis and unclog the mammary ducts by massaging the user's breast, which further improves the efficiency of milk discharge. The massage assembly can be a ball type massage assembly or a cam type massage assembly, both of which can massage the user's breast, unclog the mammary ducts, and improve the efficiency of milk discharge. It can be understood that, the massage assembly can also be a combination of the above two massage assemblies, which can perform massage on the user's breast with different effects simultaneously or in batches, further improving the efficiency of milk discharge.

In an embodiment, the physiotherapy unit 12 also includes a warm compress assembly, which performs warm compress on the user's breast to achieve a physiotherapy effect, promote metabolism and improve microcirculation, dilate small blood vessels to improve microcirculation and promote local metabolism through local thermal stimulation, thereby improving the milk discharge efficiency.

In an embodiment, the physiotherapy unit 12 also includes a cold compress assembly, which performs cold compression on the user's breast to achieve analgesic and swelling effects and prevent inflammation of the user's breast. A semiconductor cooling plate is used as the cold compress assembly. The semiconductor cooling plate can form a cold end and a warm end through electricity. The cold end is indirectly abutted against the user's breast through the breast pump shield 11 to achieve the effect of cold compress physiotherapy.

In an embodiment, the breast pump 100 further includes a power supply system electrically connected to the negative pressure assembly 32 and the physiotherapy structure for supplying power to the negative pressure assembly 32 and the physiotherapy structure.

In an embodiment, the power supply system can be rechargeable batteries, storage batteries or power cords, which is not limited here, as long as it can supply power to the negative pressure assembly 32 and the physiotherapy structure to meet functional needs.

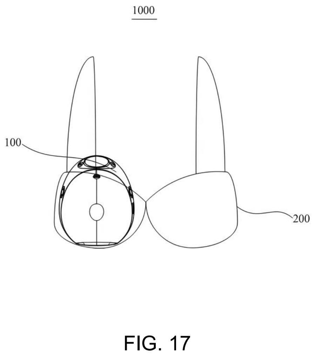

The present application also provides a breast pumping brassiere 1000, with reference to FIG. 17, including a bra main body 200 and a breast pump 100. The bra main body 200 includes a shoulder strap and a shield body, the breast pump 100 is as described above. The breast pump 100 is located on the shield body, and the breast pump shield 11 of the breast pump 100 is fitted with the breast.

In an embodiment, the bra main body 200 can be seen in daily life. The specific shape and material should be specifically selected according to the specific needs of the user. The breast pump 100 is installed in the shield body. Through the support of the shield body and the support of the shoulder strap, the breast pump 100 can be fitted with the user's breast, which can free both hands.

In related art, the bionic infant mouth structure of the breast pump 100 generally utilizes the cavity formed between the outer rigid cover of the flange 112 and the internal flexible flange 112. During negative pressure suction, the massage structure formed by the cavity can massage the breast, a shell structure of the flange 112 is required to cooperate with the flange 112 to form the massage structure, which is relatively complex. The present application provides a breast pump shield 11 with a simpler structure, which can realize the bionic infant mouth structure.

The present application also provides a breast pump shield 11, with reference to FIG. 1, FIG. 2 and FIG. 19, used in a breast pump device or a breast massage device fitted with breast, including the flange 112, a nipple accommodating portion 111 and an elastic deformation structure 1121. The flange 112 is configured for fitting with the breast, the nipple accommodating portion 111 is configured for accommodating the nipple, the flange 112 is communicated with the nipple accommodating portion 111, and the elastic deformation structure 1121 is integrated in or detachably connected to the breast pump shield 11.

In an embodiment, the breast pump shield 11 includes the flange 112 and the nipple accommodating portion 111. The nipple accommodating portion 111 is provided with openings at two ends and penetrated for accommodating the nipple. The material of the nipple accommodating portion 111 can be the rigid material, such as plastic, metal tube, and glass tubes, etc., and can also be flexible material or semi-flexible material, such as rubber or silica gel, etc., which is not limited here. The flange 112 includes a flange body and the elastic deformation structure 1121. The elastic deformation structure 1121 is made of the flexible material, such as rubber or silica gel. When negative pressure is applied to the flange 112, the elastic deformation structure 1121 can deform to massage the user's breast, promote blood circulation to remove blood stasis, unclog the mammary ducts, and further improve the efficiency of milk discharge. The flange body and the elastic deformation structure 1121 can be integrated or connected through glue or other methods.

The breast pump shield 11 includes the flange 112, the nipple channel and the elastic deformation structure 1121. The flange 112 is configured to be sealed and fitted with the breast, one side of the flange 112 fitted with the breast is flared, so that the flange 112 can be better fitted with breast to form a closed space between the flange 112 and the breast. One side of the flange 112 away from the breast is provided with an opening, and the nipple channel is communicated with the flange 112 through the opening. The nipple channel is configured to accommodate the nipple. The breast is fitted with the flange 112 to form the closed space, so that the nipple is located in the nipple channel. The elastic deformation structure 1121 is elastic and can be deformed and protruded to the inside of the flange 112 under the influence of negative pressure, thereby massaging the breast and promoting milk to flow out from the breast into the nipple channel. The negative pressure system is communicated with the inside of the breast pump shield 11. The negative pressure system is configured to extract gas from the breast pump shield 11, so that the inside of the breast pump shield 11 is in a negative pressure state, the elastic deformation structure 1121 is affected by the negative pressure and deforms to the inside of the flange 112 to generate a protrusion, and the elastic deformation structure 1121 massages the breast through the protrusion fitted with the undeformed inner wall of the flange 112.

In an embodiment, as shown in FIG. 2, the elastic deformation structure 1121 is provided at the connection between the flange 112 and the nipple accommodating portion 111.

In an embodiment, the elastic deformation structure 1121 is located at the connection between the flange 112 and the nipple accommodating portion 111, which can massage the user's mammary areola, promote blood circulation to remove blood stasis, unclog the conduit at the mammary areola, and further improve the efficiency of milk discharge.

Please referring to FIG. 2, in an embodiment, the elastic deformation structure 1121 is located at a lower position on the center line of the nipple accommodating portion 111.

In an embodiment, in order to simulate the sucking effect of infant, the elastic deformation structure 1121 is provided at the lower position of the center line of the nipple accommodating portion 111. When the flange 112 is fitted with the user's breast, the elastic deformation structure 1121 is located at the lower part of the user's breast. At this time, the negative pressure is applied to the flange 112, the elastic deformation structure 1121 will bulge, thereby simulating the peristaltic effect of the infant's mouth and jaw, further improving the efficiency of milk discharge.

In an embodiment, a plurality of elastic deformation structures 1121 are provided, and the plurality of elastic deformation structures 1121 are evenly distributed along the circumferential direction of the flange main body 1122.

In an embodiment, the plurality of elastic deformation structure 1121 are provided on the flange 112. The plurality of elastic deformation structures 1121 are provided at intervals along the circumferential direction of the flange main body 1122, which can massage the user's breast in multiple places at the same time, further improving the efficiency of milk discharge.

In an embodiment, the flange 112 is made of flexible material, and the thickness of the flange main body 1122 is greater than the thickness of the elastic deformation structure 1121.

In an embodiment, the flange 112 is made of flexible material, which can improve the user's comfort. At this time, the flange main body 1122 and the elastic deformation structure 1121 are integrated to improve durability. The thickness of flange main body 1122 is greater than the thickness of elastic deformation structure 1121. When negative pressure is applied to the flange 112, the deformation amount of the elastic deformation structure 1121 is greater than the deformation amount of the flange main body 1122, and the elastic deformation structure 1121 can protrude from flange main body 1122 to massage the user's breast, further improving the efficiency of milk discharge.

In an embodiment, the nipple accommodating portion 111 is made of rigid material.

In an embodiment, the nipple accommodating portion 111 is made of rigid material, such as plastic, metal tube, glass tube, etc. When negative pressure is applied to the flange 112, the nipple accommodating portion 111 will not deform due to the negative pressure, which may otherwise cause damage to the user's nipples.

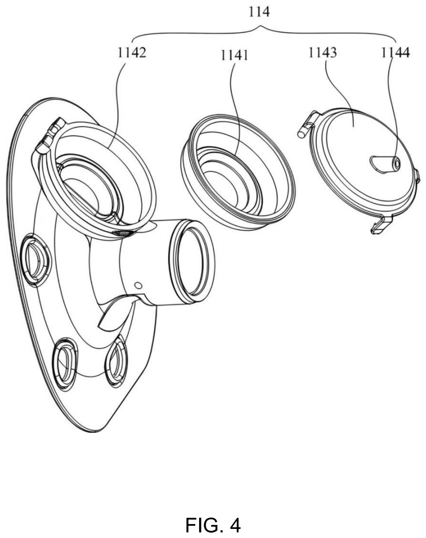

In an embodiment, with reference to FIG. 4, the breast pump shield 11 also includes a diaphragm bracket 114. The diaphragm bracket 114 is located on the breast pump shield 11 and is communicated with the breast pump shield 11. The pressure diaphragm 1141 is located on the diaphragm bracket 114.

In an embodiment, the breast pump shield 11 is provided with a diaphragm bracket 114, the diaphragm bracket 114 is provided with a pressure diaphragm 1141, the diaphragm bracket 114 is communicated with the breast pump shield 11, and the negative pressure assembly 32 can indirectly apply the negative pressure to the breast pump shield 11 through the pressure diaphragm 1141, so as to deform the elastic deformation structure 1121 to massage the user's breast.

The present application also provides a breast pump system, with reference to FIG. 1, FIG. 2, FIG. 5 and FIG. 8, including the breast pump shield 11, the negative pressure assembly 32 and the milk storage assembly 20. The breast pump shield 11 is as described above and is configured for fitting with breast, the negative pressure assembly 32 is configured for applying negative pressure to the breast pump shield 11 to make the elastic deformation structure 1121 bulge inward, and the milk storage assembly 20 is communicated with the breast pump shield 11 so that the milk flows into the milk storage assembly 20.

The present application also provides a breast pump 100, with reference to FIG. 1 and FIG. 8, including the host 30 and a breast pump system, and the host 30 is provided with an accommodating chamber. The breast pump system is as described above. The flange 112 of the breast pump shield 11 is connected to one surface of the host 30, the milk storage assembly 20 is connected to the other surface of the host 30 away from the flange 112, and the negative pressure assembly 32 is at least partially located in the accommodating chamber in the host 30.

The present application also provides a breast pumping brassiere 1000, with reference to FIG. 17, including a bra main body 200 and the breast pump 100. The bra main body 200 includes a shoulder strap and a shield body. The breast pump 100 is as described above and is located in the shield body, and the breast pump shield 11 of the breast pump 100 is fitted with the breast.

In an embodiment, the breast pump 100 is provided with a fixing portion, and the fixing portion is detachably connected to the bra main body 200 to fix the breast pump 100 to the bra main body 200.

In an embodiment, the fixing portion is provided on the breast pump 100 and is detachably connected to the bra main body 200. The fixing portion can be an assembly such as a clip or a hook-and-loop fastener, which is not limited here, which can prevent the breast pump 100 from falling from the bra main body 200 during use to cause accidents.

In an embodiment, the fixing portion is a clip, and the clip is located at the host 30 of the breast pump 100.

In an embodiment, the clip is used as the fixing portion, and the clip can be snapped with the bra main body 200 to facilitate disassembly and improve connection stability.

The present application also provides a breast pump 100, with reference to FIG. 1, including the breast pump shield 11, the host 30 and the milk storage assembly 20. The breast pump shield 11 includes the flange 112 and the nipple accommodating portion 111. The flange 112 is configured for fitting with the breast, the nipple accommodating portion 111 is provided with openings at both ends and is penetrated for accommodating the nipple, and the flange 112 is connected to one end of nipple accommodating portion 111. The host 30 includes a housing 31, the accommodating chamber is inside the housing 31, a through avoiding hole is provided at the middle of the housing 31, the other opening of the nipple accommodating portion 111 is provided in the avoiding hole, and the negative pressure assembly 32 is at least partially provided in the accommodating chamber. The negative pressure assembly 32 is configured to directly or indirectly apply negative pressure to the nipple accommodating portion 111. The milk storage assembly 20 is detachably connected to the host 30 and is communicated with the other opening of the nipple accommodating portion 111.

In an embodiment, the breast pump 100 includes the breast pump shield 11, the host 30 and the milk storage assembly 20. The through avoiding hole is provided at the middle of the housing 31 of the host 30. The nipple accommodating portion 111 of the breast pump shield 11 can pass through the avoiding hole and is communicated with the milk storage assembly 20, and the breast pump shield 11 and the milk storage assembly 20 are respectively arranged on opposite sides of the main body. The integrated design can reduce the volume of the breast pump 100, which is easy to carry and use. The housing 31 is provided with the accommodating chamber, and the negative pressure assembly 32 is arranged inside the accommodating chamber. The negative pressure assembly 32 may include an air pump, a solenoid valve, and an air path. The air pump may be one or more of a diaphragm pump, a piezoelectric pump, and a piston pump. The number can be one or more. The negative pressure assembly 32 can directly communicate with the breast pump shield 11 through the air path or indirectly communicate with the breast pump shield 11 through the diaphragm or other means to apply negative pressure to the breast pump shield 11 to deform the breast pump shield 11 to massage the breast, thereby achieving the breast pump. The sucked milk can flow into the milk storage assembly 20 through the nipple accommodating portion 111 to achieve temporary storage of milk. It can be understood that, in an embodiment, the negative pressure assembly 32 can also be a mechanical pump or a hydraulic pump, as long as it can drive the breast pump shield 11 to generate negative pressure.

In an embodiment, with reference to FIG. 4, the breast pump 100 further includes a pressure diaphragm 1141. The pressure diaphragm 1141 is provided between the housing 31 and the breast pump shield 11. The negative pressure assembly 32 applies negative pressure to the breast pump shield 11 through the pressure diaphragm 1141.

In an embodiment, the negative pressure assembly 32 indirectly applies negative pressure on the breast pump shield 11 through the pressure diaphragm 1141 sandwiched between the housing 31 and the breast pump shield 11, causing the breast pump shield 11 to deform and massage the breast, thereby achieving the effect of breast pump. The sucked milk can flow into the milk storage assembly 20 through the nipple accommodating portion 111 to achieve temporary storage of milk.

In an embodiment, the pressure diaphragm 1141 is detachably sealed or self-sealed on the breast pump shield 11, so that when the negative pressure assembly 32 operates, the pressure diaphragm 1141 deforms and drives the breast pump shield 11 to generate negative pressure.

In an embodiment, the pressure diaphragm 1141 is detachably sealed or self-sealed and is provided on the breast pump shield 11. The negative pressure assembly 32 applies negative pressure to the pressure diaphragm 1141 and indirectly applies negative pressure to the breast pump shield 11, thereby preventing milk sucking back to the pump assembly, so as to prevent problems such as milk contamination and circuit damage.

In an embodiment, as shown in FIG. 1 and FIG. 4, the diaphragm bracket 114 is provided on one side of the breast pump shield 11 towards the host 30, the pressure diaphragm 1141 is located on the diaphragm bracket 114, and the diaphragm bracket 114 is sealingly communicated with the negative pressure assembly 32.

In an embodiment, the breast pump shield 11 is provided with the diaphragm bracket 114. The diaphragm bracket 114 is sealingly communicated with the negative pressure assembly 32. The diaphragm bracket 114 is provided with the pressure diaphragm 1141. The negative pressure assembly 32 indirectly applies negative pressure to the breast pump shield 11 by applying negative pressure to the pressure diaphragm 1141. The diaphragm bracket 114 can play a supporting role to form a sealed negative pressure space between the pressure diaphragm 1141 and the breast pump shield 11, so that the breast pump shield 11 has a better massage breast effect under the action of the pressure diaphragm 1141, improving the efficiency of milk discharge.

Please referring to FIG. 4, in an embodiment, the diaphragm bracket 114 includes a supporting frame 1142, a cover 1143 and a connecting structure 1144. The supporting frame 1142 is a bowl-shaped structure with openings at both ends. An opening of the supporting frame 1142 is communicated with the breast pump shield 11, the cover 1143 covers the other opening of the supporting frame 1142, the pressure diaphragm 1141 is sandwiched between the cover 1143 and the other opening of the supporting frame 1142, one end of the connecting structure 1144 is connected to and communicated with the cover 1143, and the other end of the connecting structure 1144 is communicated with the negative pressure assembly 32. The circumferential side of the cover 1143 is provided with a rotary connecting member and a clamping member, the rotary connecting member is rotationally connected to an edge of the other opening of the supporting frame 1142, and the clamping member is snapped to the edge of the other opening of the supporting frame 1142.

In an embodiment, the diaphragm bracket 114 is made of hard material, such as plastic, and is not easily deformed. The diaphragm bracket 114 includes the supporting frame 1142, the cover 1143 and the connecting structure 1144. The supporting frame 1142 is a bowl-shaped structure with openings at both ends. One opening is communicated with the breast pump shield 11, and the other opening is provided on the pressure diaphragm 1141. The cover 1143 covers the supporting frame 1142 and is communicated with the negative pressure assembly 32 through the connecting structure 1144, the negative pressure assembly 32 applies negative pressure to the pressure diaphragm 1141 through the connecting structure 1144 and applies negative pressure on the breast pump shield 11, and the breast pump shield 11 deforms and massages the breast, improving the efficiency of milk discharge.

The cover 1143 is detachably connected to the supporting frame 1142, making it easy to replace the pressure diaphragm 1141 that is elastically failed due to repeated deformation, and simplifying the maintenance and installation steps. One side of the cover 1143 is provided with the rotary connecting member, and the other end of the cover 1143 is provided with the clamping member. The cover 1143 is rotatably connected to the supporting frame 1142 through the rotary connecting member and is snapped with the supporting frame 1142 through the clamping member, thereby realizing a quick disassembly and assembly between the cover 1143 and the supporting frame 1142.

In an embodiment, the pressure diaphragm 1141 is sealingly connected to the housing 31.

In an embodiment, the pressure diaphragm 1141 is sealingly connected to the housing 31 of the host 30. At the same time, there is a sealed space between the housing 31 of the host 30 and the breast pump shield 11. The negative pressure assembly 32 applies negative pressure to the pressure diaphragm 1141, thereby squeezing the gas in the sealed space, which eventually deforms the breast pump shield 11 to massage the breast and improve the efficiency of milk discharge.

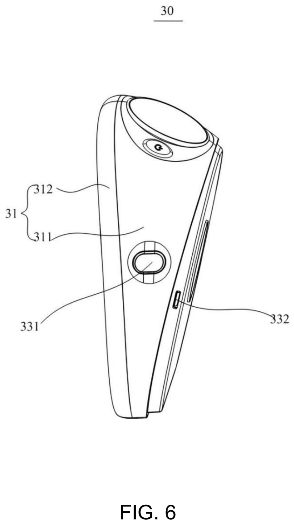

In an embodiment, with reference to FIG. 6 to FIG. 8, the gravity center of the host 30 is above the axis of the nipple accommodating portion 111.

In an embodiment, the gravity center of the host 30 is above the axis of the nipple accommodating portion 111. That is, when the user wears the breast pump 100, the center of the host 30 is located above the user's nipple, enabling the breast to support part of the weight of the host 30, which improves the stability and saves laboring.

In an embodiment, with reference to FIG. 1, FIG. 5 and FIG. 6, a surface of the housing 31 towards the milk storage assembly 20 of the host 30 is a first inclined surface, and a surface of the milk storage assembly 20 towards the host 30 is a second inclined surface. The first inclined surface is fitted with the second inclined surface.

In an embodiment, the surface of housing 31 towards the milk storage assembly 20 of the host 30 is the first inclined surface, and the surface of the milk storage assembly 20 towards host 30 is the second inclined surface. The first inclined surface is fitted with the second inclined surface to facilitate installing the milk storage assembly 20. The cross section of the milk storage assembly 20 is narrow at the top and wide at the bottom, so it can hold more milk in the same volume.

In an embodiment, as shown in FIG. 1, the milk storage assembly 20 includes a milk bowl cover 21 and a bowl body 22. The bowl body 22 is provided with a milk storing space with an opening, and the milk bowl cover 21 covers the opening. The milk bowl cover 21 is provided with a through hole, the other end of the nipple accommodating portion 111 is communicated with the bowl body 22 through the through hole, so that the milk flows into the bowl body 22.

In an embodiment, the milk bowl cover 21 and the bowl body 22 are made of food-grade environmentally friendly materials, such as polypropylene, polyethersulfone, etc. The milk bowl cover 21 is detachably connected to the bowl body 22, such as the snap connection, to facilitate the disassembly and cleaning of the milk bowl cover 21 and the bowl body 22 after use. The nipple accommodating portion 111 all passes through the through hole opened on the milk bowl cover 21 and is communicated with the milk storing space of the bowl body 22. The milk can directly flow into the bowl body 22 through the nipple accommodating portion 111 and be temporarily stored in the milk storing space. It can be understood that, in an embodiment, the nipple accommodating portion 111 can also be indirectly connected to the milk storage assembly 20, which are communicated through the pipeline, etc., so as to store the milk.

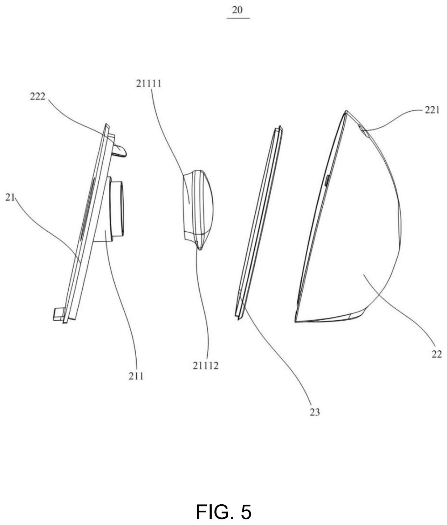

In an embodiment, referring to FIG. 1 and FIG. 5, the milk bowl cover 21 is provided with an assembly cylinder 211. One end of the assembly cylinder 211 is connected to the edge of the through hole, the assembly cylinder 211 is located in the milk storing space, the other end of the assembly cylinder 211 is provided with a milk flowing hole, and the other end of the nipple accommodating portion 111 is inserted into the assembly cylinder 211. A first sealing member 1111 is provided between the nipple accommodating portion 111 and the assembly cylinder 211. The wall of the assembly cylinder 211 extends inward the circumferential direction to form a protruding flange, and the first sealing member 1111 is provided at the other end of the nipple accommodating portion 111. The nipple accommodating portion 111 presses the first sealing member 1111 against the flange to seal, and the first sealing member 1111 is a suction cup, which not only plays the sealing function, but also forms a locking suction force between the breast pump shield 11 and the milk bowl, making the two more closely combined.

In an embodiment, in order to improve the connection strength between the nipple accommodating portion 111 and the milk storage assembly 20, the milk bowl cover 21 extends toward the bowl body 22 at the through hole to form an assembly cylinder 211, and a milk flowing hole is opened at one end of the assembly cylinder 211. The other end of the nipple accommodating portion 111 is directly inserted into the assembly cylinder 211, and the connection is more stable. The milk passes through the nipple accommodating portion 111 and the milk flowing hole, and finally flows into the milk storing space.

The first sealing member 1111 is provided between the nipple accommodating portion 111 and the assembly cylinder 211. A sealing groove in the circumferential direction can be opened on the outer peripheral wall of the nipple accommodating portion 111. The first sealing member 1111 is arranged in the sealing groove. The nipple accommodating portion 111 is abutted against the first sealing member 1111 on the inner wall of the assembly cylinder 211 to seal and prevent milk from overflowing or backflowing.

The inner wall circumferential direction of the assembly cylinder 211 protrudes inward to form the protruding flange, and the first sealing member 1111 is sandwiched between the other end surface of the nipple accommodating portion 111 and the protruding flange, thereby forming a sealed structure to prevent milk from overflowing or backflowing.

The first sealing member 1111 is the suction cup, and the suction nozzle of the suction cup should be set toward the direction of the protruding flange. The end surface of the nipple accommodating portion 111 can press the suction cup and be adsorbed on the surface of the protruding flange towards the nipple accommodating portion 111, so as to seal. At the same time, the connection strength between the host 30 and the milk storage assembly 20 can be increased, further preventing the milk from overflowing or backflowing, and preventing the milk storage assembly 20 from falling off the host 30.

In an embodiment, referring to FIG. 5, the milk bowl cover 21 is detachably connected to the bowl body 22. A second sealing member 23 is provided between the milk bowl cover 21 and the bowl body 22. The second sealing member 23 is a flexible or semi-flexible sealing ring. The second sealing member 23 is integrated in one of the milk bowl cover 21 or the bowl body 22, and is sealingly connected to the other of the milk bowl cover 21 or the bowl body 22, or the second sealing member 23 is detachably connected between the milk bowl cover 21 and the bowl body 22.

In an embodiment, the milk bowl cover 21 is detachably connected to the bowl body 22, such as the snap connection, to facilitate the disassembly and cleaning of the milk bowl cover 21 and the bowl body 22 after use. The second sealing member 23 is provided between the milk bowl cover 21 and the bowl body 22. A first limiting circle is provided on the opening edge of the bowl body 22. A second limiting circle is formed on one side of the milk bowl cover 21 towards the bowl body 22. The second sealing member 23 is formed between the first limiting circle and the second limiting circle. The second sealing member 23 improves the sealing property of the connection between the milk bowl cover 21 and the bowl body 22, preventing milk from flowing out from the connection, which may otherwise cause waste or further contaminate the remaining milk in the milk storing space after the milk flows out. The second sealing member 23 is a flexible or semi-flexible sealed ring. It can be understood that, in an embodiment, a sealed suction cup, etc. can also be used. The second sealing member 23 can also be integrated with one of the milk bowl cover 21 or the bowl body 22, and sealingly connected to the other one of the milk bowl cover 21 or the bowl body 22, so as to seal between the milk bowl cover 21 and the bowl body 22 to prevent the milk from leaking out. The integrated configuration can facilitate the installation, disassembly and cleaning of the second sealing member 23.

In an embodiment, as shown in FIG. 5, the milk storage assembly 20 also includes a check valve 2111. The check valve 2111 is located at the other end of the assembly cylinder 211 and is communicated with the milk flowing hole to prevent the milk in the bowl body 22 from flowing back. The check valve 2111 includes a connecting cylinder 21111, one end of the connecting cylinder 21111 is connected to the assembly cylinder 211, the other end of the connecting cylinder 21111 extends downward to form a duckbill valve end 21112. One side of the connecting cylinder 21111 away from duckbill valve end 21112 extends outward to form a buckle portion.

In an embodiment, the other end of the assembly cylinder 211 towards the bowl body 22 is detachably connected with the check valve 2111 configured to guide the milk from the assembly cylinder 211 into the milk storage chamber in a single direction, which prevents the milk from flowing backward into the assembly cylinder 211 and improves the milk collection efficiency. The connecting cylinder 21111 is configured to connect with the assembly cylinder 211, and the other end of the connecting cylinder 21111 extends downward to form the duckbill valve end 21112, which makes the milk flow out in a single direction without flowing back. One side of the connecting cylinder 21111 away from duckbill valve end 21112 protrudes outward to form the buckle portion, which is convenient for the user to hold to remove the connecting cylinder 21111 from the assembly cylinder 211 for cleaning or replacement.

In an embodiment, as shown in FIG. 5, the bowl body 22 is provided with a milk pouring hole 221 near the top, and the milk pouring hole 221 is communicated with the milk storing space. One side of the milk bowl cover 21 towards the bowl body 22 is provided with a baffle 222, and the baffle 222 is set corresponding to the milk pouring hole 221.

In an embodiment, the milk pouring hole 221 communicated with the milk storing space is provided near the top of the bowl body 22, which can be configured to discharge air. When necessary, the milk can be poured directly from the milk pouring hole 221. The baffle 222 blocks the milk pouring hole 221. When the milk in the milk storage assembly 20 needs to be poured into the storage bag, the baffle 222 acts as a diversion to prevent the milk from instantly covering the milk pouring hole 221, which may otherwise cause the negative pressure to form in the milk storage assembly 20 and the milk can not be poured smoothly. At the same time, when the milk in the milk storage assembly 20 is full, the liquid level will shake due to the user's activities, and the baffle 222 can reduce the amplitude of the liquid level shaking to prevent milk from spilling from the milk pouring hole 221. In addition, the baffle 222 can also prevent dirt from falling into the milk storage chamber from the milk pouring hole 221. Of course, it can be understood that the position of the baffle 222 is not limited to being set on the milk bowl cover 21, it can also be set on the corresponding position of the bowl body 22, or it can be set respectively on the milk bowl cover 21 and the bowl body 22. When the assembly is completed, a complete baffle 222 structure is formed. As long as the baffle 222 can divert milk when pouring milk from the milk pouring hole 221, the specific position, size and shape of the baffle 222 can be customized according to actual needs.

Furthermore, in order to prevent the milk storage assembly 20 removed from the host 30 from tipping over and causing the milk to flow out of the milk pouring hole or when it is too late to pour the milk out of the milk storage assembly 20, a removable rubber plug is provided in the milk pouring hole. The rubber plug can block the milk pouring hole, so that the milk can be stored in the milk storage assembly 20 for a short time and prevent the milk from flowing out when the milk storage assembly 20 is dumped. The rubber plug is also made of environmentally friendly, food-grade silica gel material.

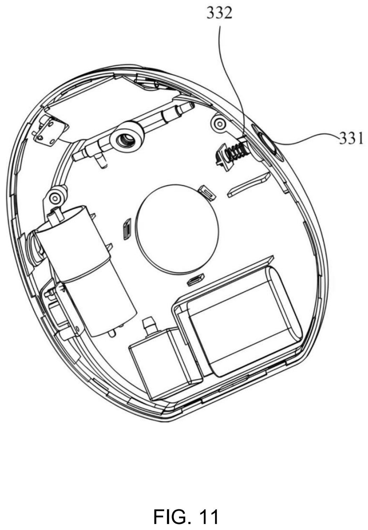

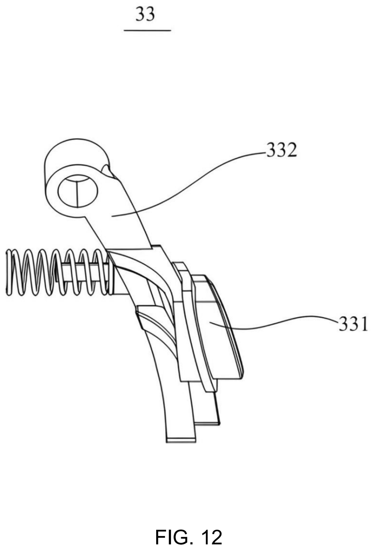

In an embodiment, referring to FIG. 11 and FIG. 12, the detachable structure 33 is provided between the host 30 and the milk storage assembly 20 to realize the detachable connection between the host 30 and the milk storage assembly 20. The detachable structure 33 includes an unlock key 331 and a lock structure 332. The lock structure 332 has a locked state that is connected to the host 30 and the milk storage assembly 20, and an unlocked state that is disconnected from the host 30 and the milk storage assembly 20. The unlock key 331 is connected to the lock structure 332 and can control the lock structure 332 to switch from the locked state to the unlocked state. The unlock key 331 is one of the pressing key, the pulling key, the sliding key and the rotating key.

In an embodiment, the detachable structure 33 is used between the host 30 and the milk storage assembly 20 to achieve a detachable connection. The milk storage assembly 20 can be quickly removed or installed from the host 30, which facilitates operation and saves the user's time. The lock structure 332 can integratedly connect the host 30 and the milk storage assembly 20, and the unlock key 331 can control the state of the lock structure 332 to switch from the locked state of connecting the host 30 and the milk storage assembly 20 to the unlocked state of disconnecting the host 30 and the milk storage assembly 20, which enables the quick removal or installation of the milk storage assembly 20. The unlock key 331 can be the pressing key, the pulling key, the sliding key or the rotating key. The specific style can be selected according to the state switching method of the lock structure 332 or the actual needs of the product, which is not limited here.

In an embodiment, the detachable structure 33 includes a first magnetic portion provided on the host 30 and a second magnetic portion provided on the milk storage assembly 20, the first magnetic portion is magnetically connected to the second magnetic portion.

In an embodiment, the detachable connection between the host 30 and the milk storage assembly 20 is achieved by magnetically connecting the first magnetic portion and the second magnetic portion respectively provided on the host 30 and the milk storage assembly 20, ultimately realizing quick disassembly or installation. The milk storage assembly 20 is convenient for user operation and saves the user's time.

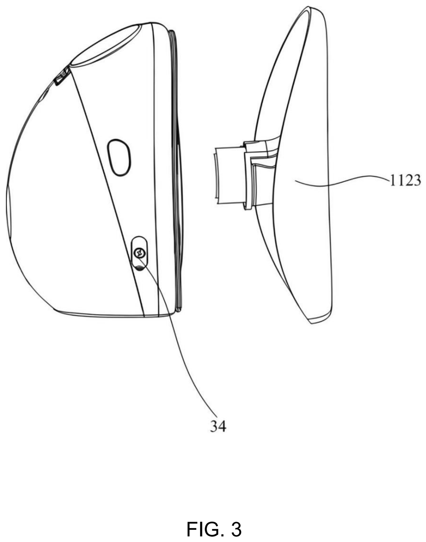

In an embodiment, with reference to FIG. 3, the outer edge of the breast pump shield 11 extends outward and is folded to form a folding portion 1123, and the folding portion 1123 covers at least part of the housing 31 of the host 30.

In an embodiment, the outer edge of the breast pump shield 11 extends outward and is folded to form the folding portion 1123. The folding portion 1123 can cover at least part of the housing 31 of the host 30, play a role in protecting the housing 31, and can play a role in shock-absorbing and anti-fall.

Referring to FIG. 3, in an embodiment, the host 30 includes a charging hole 34, and the folding portion 1123 covers the charging hole 34.

In an embodiment, when the flange 112 is fastened and installed on the host 30, the folding portion 1123 is provided at the circumferential direction edge of the flange main body 1122 and can cover the charging hole 34 on the host 30 when the flange 112 is fastened on the host 30. In this way, when in use, the flange 112 can seal the charging hole 34 of the host 30 to prevent liquid or dust from entering the charging hole 34, thus protecting the host 30.

In an embodiment, the folding portion 1123 wraps the entire peripheral wall of the housing 31.

In an embodiment, the folding portion 1123 can cover the entire outer peripheral wall of the housing 31 of the host 30 to protect the housing 31 and improve the shockproof and fall-proof performance of the host 30.

The present application also provides a breast pump system, with reference to FIG. 1, FIG. 8 and FIG. 9, including the breast pump shield 11, the milk storage assembly 20, the host 30, the power supply system 50 and the remote control 40. The breast pump shield 11 is configured for fitting with breast, the milk storage assembly 20 is communicated with the breast pump shield 11 for receiving and storing milk, the host 30 includes the housing 31, the housing is provided with the accommodating chamber, the negative pressure assembly 32 is provided in the accommodating chamber, and the negative pressure assembly 32 is configured to apply negative pressure to the milk storage assembly 20 or the breast pump shield 11. The power supply system 50 is located in the accommodating chamber and is electrically connected to the negative pressure assembly 32, the remote control 40 is configured to communicate with the negative pressure assembly 32 to operate or stop the negative pressure assembly 32. The remote control 40 includes a housing of the remote control and a control circuit board. The housing of the remote control is provided with a cavity body, and the control circuit board is located in the cavity body. The housing of the remote control is provided with a control panel. The control panel is electrically connected to the control circuit board, and the remote control 40 is detachably located on the outer wall of the housing 31 of the host 30 or the outer wall of the milk storage assembly 20.

In an embodiment, the remote control 40 is communicated with the negative pressure assembly 32 and can operate or stop the negative pressure assembly 32. The housing of the remote control is provided with the control panel, and the control circuit board in the remote control 40 can be directly controlled through the control panel. When the user does not need to use the breast pump 100, the remote control 40 is connected to the outer wall of the host 30 or the outer wall of the milk storage assembly 20 to realize the storage of the remote control 40, so as to avoid difficulty in finding the remote control 40 when the user needs to use it.

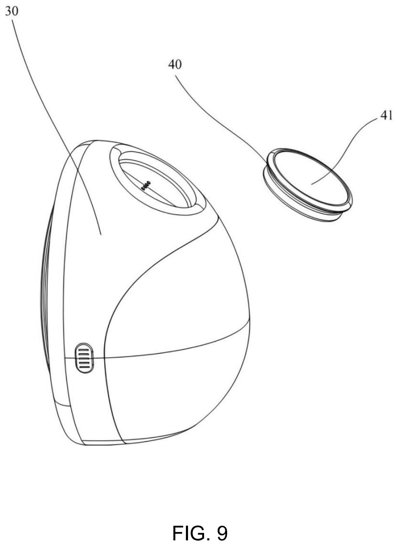

In an embodiment, with reference to FIG. 9, an accommodating groove is opened on the top of the housing, and the remote control 40 is accommodated in the accommodating groove and detachably connected to the inner wall of the accommodating groove.

In an embodiment, the accommodating groove is opened at the top of the housing of the host 30, and the remote control 40 is accommodated in the accommodating groove and detachably connected to the inner wall of the accommodating groove. In general, when placing the breast pump 100, the bottom of the host 30 is in contact with the placement surface, and users usually hold both sides of the host 30 when using it. Therefore, setting the accommodating groove on the top of the host 30 is more in line with the user's usage habits. The accommodating groove can provide an orderly storage space for the remote control 40, and can also prevent the remote control 40 from colliding with external objects and falling. It can also make full use of the space occupied by the host 30 and reduce the overall size of the breast pump 100, which takes up less space and makes the structure more compact.

In an embodiment, the breast pump system further includes a third magnetic portion and a fourth magnetic portion magnetically attracted to the third magnetic portion. The third magnetic portion and the fourth magnetic portion are respectively located on the housing of the remote control 40 and the outer wall of the housing.

In an embodiment, the breast pump 100 further includes the third magnetic portion and the fourth magnetic portion magnetically attracted to the third magnetic portion. The third magnetic portion and the fourth magnetic portion are respectively located on the housing of the remote control 40 and the housing of the host 30. The magnetic suction is configured to realize the detachable connection between the remote control 40 and the inner wall of the housing of the host 30. The remote control 40 can be quickly attached to the housing of the host, which is convenient, fast and flexible. It can be understood that, in order to further improve the connection strength, the fourth magnetic portion can be provided on the wall of the accommodating groove.

In an embodiment, as shown in FIG. 9 and FIG. 16, the remote control 40 can be disassembled from the housing of the host 30 and connected to the power supply system 50 for power supply, and the connection method between the remote control 40 and the power supply system 50 is one of metal contact connection, wireless connection or wired connection. The remote control 40 can be connected to the host 30 by the metal contact connection, the wireless connection or the wired connection to realize the power supply from host 30 to remote control 40. The remote control 40 also includes a display screen 41 provided with a housing of the remote control and is electrically connected to the control circuit board. The remote control 40 also includes a watch strap 42 configured to connect to the housing of the remote control.

In an embodiment, the power supply system 50 can provide power to the remote control 40 so that the remote control 40 does not need to be charged separately, which is convenient for the user. The remote control 40 and the host 30 can be connected by metal contact connection, wireless connection or wired connection to realize the power supply from the host 30 to the remote control 40. The housing of the remote control is provided with a display screen 41, which can more intuitively display the usage of the host 30, such as displaying the remaining power or the working status of the negative pressure assembly 32, etc., for the convenience of the user. When the remote control 40 is disassembled from the host 30, the watch strap 42 is connected to both sides of the remote control 40 to wear it on the user's arm, which can be operated at any time when needed and is not easy to lose. The watch strap 42 is detachably connected to the remote control 40. Threaded holes can be provided on both sides of the remote control 40, threaded columns are provided on both sides of the watch strap 42, and the threaded columns are screwed to the threaded holes to realize the connection between the watch strap 42 and the remote control 40, or a connecting portion can be set on the remote control 40, a through hole is opened on the connecting portion, and the watch strap 42 is passed through the through hole to realize the connection between the watch strap 42 and the remote control 40, which is not limited in the present application.

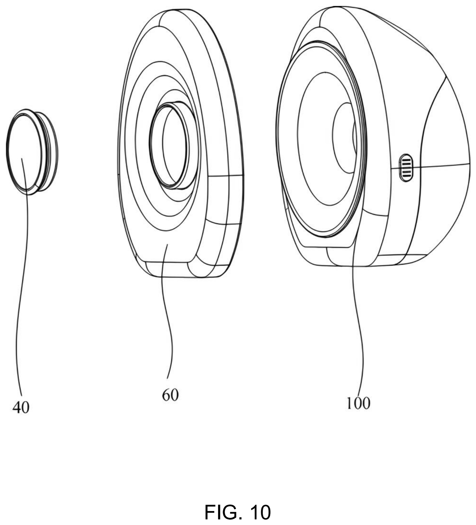

In an embodiment, with reference to FIG. 10, the milk storage assembly 20 also includes a dust cover 60. The dust cover 60 is detachably connected to the flange 112 of the breast pump shield 11. The remote control 40 is detachably located on the dust cover 60. A convex plate is formed at a middle position of the dust cover 60, and the remote control 40 is snapped to the convex plate to connect the remote control 40 to the dust cover 60.

In an embodiment, the dust cover 60 is detachably connected to the flange 112 and can completely cover the flange 112 to prevent the breast pump 100 from being dusted or polluted when idle, which may otherwise cause potential safety hazards. The remote control 40 can be provided on the dust cover 60 to store the remote control 40. The convex plate is formed at the middle of the dust cover 60. The remote control 40 is snapped in the convex plate, and the remote control 40 is connected to the dust cover 60 to store the remote control 40.

The connection relationship between the breast pump shield 11, the housing 31 and the milk storage assembly 20 described in the present application includes but is not limited to the following ways:
The breast pump shield 11 and the milk storage assembly 20 are integrated, and integrated and locked with the housing 31 to form an integral wearing unit or at least partially worn in the bra;
or the breast pump shield 11 and the milk storage assembly 20 are respectively integrated and locked with the housing 31 to form an integral wearing unit or at least partially worn in the bra.

The milk storage assembly 20 described in the present application may be one or more of a milk bowl, a milk bottle or a milk bag. The breast pump shield 11 can be directly communicated with the milk storage assembly 20, or can be communicated with other flow path through the pipe.

The power structure configured to generate negative pressure in the negative pressure assembly 32 of the breast pump 100 of the present application includes but is not limited to piezoelectric pumps, diaphragm pumps, hydraulic pumps, mechanical pumps, etc. It may be directly communicated to cause negative pressure inside the breast pump shield 11, or the negative pressure can be transmitted to a liquid barrier such as a diaphragm or an air bag to indirectly cause negative pressure inside the breast pump shield 11. The milk is introduced into the milk storage container for storage through negative pressure.

The host 30 of the breast pump 100 of the present application includes the housing 31. The wall of the host 30 is the surface of the housing 31. The negative pressure assembly 32 is at least partially installed in the housing 31. The housing 31 may also include the batter, the negative pressure air path, the circuit board, the solenoid valve and other components.

The above descriptions are only embodiments of the present application, and those skilled in the art should know that the present application is not limited to the above embodiments. The above embodiments are only examples, and embodiments that have essentially the same composition as the technical idea and play the same role and effect within the scope of the present application are included in the scope of the present application. In addition, without deviating the subject matter of the present application, other methods of applying to the mode of embodiment such variations as may be thought of by those skilled in the art, or of combining some of the constituent elements of the mode of embodiment, shall also be included in the scope of the present application.

## Claims

1. A physiotherapy structure, applied to a breast pump, **characterized by** comprising:
a breast pump shield provided with a first side contacting a breast and a second side not contacting the breast;
a physiotherapy unit abutted against or detachably connected to the second side of the breast pump shield; and
an electronic control system electrically connected to the physiotherapy unit.

2. The physiotherapy structure of claim 1, wherein the breast pump shield comprises a nipple accommodating portion and a flange, and two ends of the nipple accommodating portion are provided with openings and are penetrated; the flange is connected to an opening of the nipple accommodating portion, and the flange is configured to abut against the breast.

3. The physiotherapy structure of claim 2, wherein the flange is made of a flexible material or at least partially made of the flexible material.

4. The physiotherapy structure of claim 1, wherein the physiotherapy unit comprises at least one of a vibration member, a kneading member, a cold compress member or a warm compress member.

5. A breast pump capable of applying a physiotherapy action to a breast, **characterized by** comprising:
the physiotherapy structure of any one of claims 2 to 4;
a host comprising a housing, wherein the housing is connected to the second side of the breast pump shield or at least partially connected to the breast pump shield; the physiotherapy unit is provided on a surface of the housing towards the breast pump shield, and the physiotherapy unit is configured to apply the physiotherapy action to the breast through the breast pump shield;
a negative pressure assembly at least partially provided in the housing; and
a milk storage assembly communicated with the breast pump shield;
wherein the electronic control system is electrically connected to the negative pressure assembly.

6. The breast pump of claim 5, wherein the physiotherapy unit comprises a vibration motor configured to vibratingly massage the breast.

7. The breast pump of claim 6, wherein a plurality of vibration motors are provided, and the plurality of the vibration motors are provided at intervals along a circumferential direction of the flange or the plurality of the vibration motors are scatterly distributed with the flange as a center.

8. The breast pump of claim 6, wherein the vibration motor is protruded from one surface of the host towards the breast pump shield, and one surface of the flange towards the host is provided with a recessed groove; a recessed direction of the recessed groove is away from the host, and the vibration motor is at least partially accommodated in the recessed groove; and
at least a bottom of the recessed groove and/or at least a sidewall of the recessed groove are made of the flexible material, and the vibration motor is abutted against the bottom of the recessed groove; the sidewall of the recessed groove is sleeved on at least a part of a structure of the vibration motor, and one side surface of the flange near the breast is flat.

9. The breast pump of claim 8, wherein the flange comprises a flange main body, and the recessed groove is integrated or detachably fixed on the flange main body.

10. The breast pump of claim 9, wherein the flange main body is made of a rigid material, and at least part of the recessed groove is made of the flexible material and integrated on the flange main body; or
the flange main body and the recessed groove are integrated, and are made of the flexible material or a semi-flexible material.

11. The breast pump of any one of claims 5 to 10, further comprising:
a power supply system electrically connected to the negative pressure assembly and the physiotherapy structure and configured to provide a power to the negative pressure assembly and the physiotherapy structure.

12. A breast pumping brassiere, **characterized by** comprising:
a bra main body comprising a shoulder strap and a shield body; and
the breast pump of any one of claims 5 to 11;
wherein the breast pump is provided in the shield body, and the breast pump shield of the breast pump is fitted with the breast.

13. A breast pump shield, applied in a breast pump device or a breast massage device to be fitted with the breast, **characterized by** comprising:
a flange;
a nipple accommodating portion; and
an elastic deformation structure;
wherein the flange is configured to fit with the breast, and the nipple accommodating portion is configured for accommodating nipples; the flange is communicated with the nipple accommodating portion, and the elastic deformation structure is integrated with or detachably connected with the breast pump shield.

14. The breast pump shield of claim 13, wherein the elastic deformation structure is provided at a connection between the flange and the nipple accommodating portion.

15. The breast pump shield of claim 14, wherein the elastic deformation structure is provided below a midline of the nipple accommodating portion.

16. The breast pump shield of claim 13, wherein a plurality of elastic deformation structures are provided, and the plurality of the elastic deformation structures are evenly distributed along a circumferential direction of the flange.

17. The breast pump shield of claim 13, wherein the flange is made of the flexible material, and a thickness of the flange is greater than a thickness of the elastic deformation structure.

18. The breast pump shield of claim 13, wherein the nipple accommodating portion is made of the rigid material.

19. The breast pump shield of claim 13, further comprising:
a diaphragm bracket;
wherein the diaphragm bracket is provided on the breast pump shield and is communicated with the breast pump shield, and a pressure diaphragm is provided on the diaphragm bracket.

20. A breast pump system, **characterized by** comprising:
the breast pump shield of any one of claims 13 to 19 configured for fitting with the breast;
a negative pressure assembly configured to apply a negative pressure to the breast pump shield to make the elastic deformation structure bulge inward; and
a milk storage assembly communicated with the breast pump shield so that the milk flows into the milk storage assembly.

21. A breast pump, **characterized by** comprising:
a host provided with an accommodating chamber; and
the breast pump system of claim 20;
wherein the flange of the breast pump shield is connected to one surface of the host; the milk storage assembly is connected to the other surface of the host away from the flange, and the negative pressure assembly is at least partially provided in the accommodating chamber in the host.

22. A breast pumping brassiere, **characterized by** comprising:
a bra main body comprising a shoulder strap and a shield body; and
the breast pump of claim 21;
wherein the breast pump is provided on the shield body, and the breast pump shield of the breast pump is fitted with the breast.

23. The breast pumping brassiere of claim 22, wherein a fixing portion is provided on the breast pump, and the fixing portion is detachably connected to the bra main body to fix the breast pump to the bra main body.

24. The breast pumping brassiere of claim 23, wherein the fixing portion is a clip provided at the host of the breast pump.

25. A breast pump, **characterized by** comprising:
a breast pump shield comprising a flange and a nipple accommodating portion, wherein the flange is configured for fitting with a breast; two ends of the nipple accommodating portion are provided with openings and penetrated for accommodating nipples, and the flange is connected to one end of the nipple accommodating portion;
a host comprising a housing, wherein an accommodating chamber is provided in the housing, and a through avoiding hole is provided at a middle of the housing; the other opening of the nipple accommodating portion is penetrated through the avoiding hole; a negative pressure assembly is at least partially provided in the accommodating chamber, and the negative pressure assembly is configured to directly or indirectly apply a negative pressure to the nipple accommodating portion; and
a milk storage assembly detachably connected to the host and communicated with the other opening of the nipple accommodating portion.

26. The breast pump of claim 25, further comprising:
a pressure diaphragm provided between the housing and the breast pump shield;
wherein the negative pressure assembly is configured to apply the negative pressure to the breast pump shield through the pressure diaphragm.

27. The breast pump of claim 26, wherein the pressure diaphragm is detachably sealed or self-sealed and is provided on the breast pump shield, so that in response to that the negative pressure assembly moves, the pressure diaphragm is configured to deform to generate the negative pressure in the breast pump shield.

28. The breast pump of claim 26, wherein a diaphragm bracket is provided on one side of the breast pump shield towards the host; the pressure diaphragm is provided on the diaphragm bracket, and the diaphragm bracket is sealingly communicated with the negative pressure assembly.

29. The breast pump of claim 28, wherein the diaphragm bracket comprises a supporting frame, a cover and a connecting structure, and the supporting frame is a bowl-shaped structure with openings at both ends; an opening of the supporting frame is communicated with the breast pump shield, and the cover is configured to cover the other opening of the supporting frame; the pressure diaphragm is sandwiched between the cover and the other opening of the supporting frame; one end of the connecting structure is connected and communicated with the cover, and the other end of the connecting structure is communicated with the negative pressure assembly; and
a rotary connecting member and a clamping member are provided on a peripheral side of the cover; the rotary connecting member is rotationally connected to an edge of the other opening of the supporting frame, and the clamping member is snap-connected to the edge of the other opening of the supporting frame.

30. The breast pump of claim 26, wherein the pressure diaphragm is sealingly connected to the housing.

31. The breast pump of claim 25, wherein a gravity center of the host is above an axis of the nipple accommodating portion.

32. The breast pump of claim 25, wherein one surface of the housing of the host towards the milk storage assembly is a first inclined surface, and one surface of the milk storage assembly towards the host is a second inclined surface; the first inclined surface is fitted with the second inclined surface.

33. The breast pump of claim 25, wherein the milk storage assembly comprises a milk bowl cover and a bowl body; the bowl body is provided with a milk storing space with an opening, and the milk bowl cover is configured to cover the opening; the milk bowl cover is provided with a through hole, and the other end of the nipple accommodating portion is communicated with the bowl body through the through hole, so that the milk flows into the bowl body.

34. The breast pump of claim 33, wherein an assembly cylinder is provided on the milk bowl cover; one end of the assembly cylinder is connected to an edge of the through hole; the assembly cylinder is provided in the milk storing space, and the other end of the assembly cylinder is provided with a milk flowing hole; the other end of the nipple accommodating portion is penetrated through the assembly cylinder;
a first sealing member is provided between the nipple accommodating portion and the assembly cylinder;
a sidewall of the assembly cylinder is configured to extend inward in a circumferential direction to form a protruding flange; the other end of the nipple accommodating portion is provided with the first sealing member, and the nipple accommodating portion is configured to press the first sealing member against the protruding flange to seal; and
the first sealing member is a suction cup.

35. The breast pump of claim 33, wherein the milk bowl cover is detachably connected to the bowl body;
a second sealing member is provided between the milk bowl cover and the bowl body;
the second sealing member is a flexible or semi-flexible sealing ring;
the second sealing member is integrated in one of the milk bowl cover and the bowl body, and is sealingly connected to the other one of the milk bowl cover and the bowl body; or
the second sealing member is detachably connected between the milk bowl cover and the bowl body.

36. The breast pump of claim 34, wherein the milk storage assembly further comprises a check valve provided at the other end of the assembly cylinder and is communicated with the milk flowing hole to prevent the milk in the bowl body from flowing back;
the check valve comprises a connecting cylinder; one end of the connecting cylinder is connected to the assembly cylinder, and the other end of the connecting cylinder is configured to extend downward to form a duckbill valve end; and
one side of the connecting cylinder away from the duckbill valve end is configured to extend outward to form a buckle portion.

37. The breast pump of claim 33, wherein a milk pouring hole is provided near a top of the bowl body, and the milk pouring hole is communicated with the milk storing space; and
one side of the milk bowl cover towards the bowl body is provided with a baffle, and the baffle is provided relative to the milk pouring hole.

38. The breast pump of claim 35, wherein a detachable structure is provided between the host and the milk storage assembly so that the host is detachably connected to the milk storage assembly;
the detachable structure comprises an unlock key and a lock structure; the lock structure has a locked state that is snap-connected to the host and the milk storage assembly and an unlocked state that is separated from the host and the milk storage assembly, and the unlock key is connected to the lock structure and is configured to control the lock structure to switch from the locked state to the unlocked state; and
the unlock key is one of a pressing key, a pulling key, a sliding key and a rotating key.

39. The breast pump of claim 38, wherein the detachable structure comprises a first magnetic portion provided on the host and a second magnetic portion provided on the milk storage assembly, and the first magnetic portion is magnetically connected to the second magnetic portion.

40. The breast pump of claim 35, wherein an outer edge of the breast pump shield is configured to extend outward and is folded to form a folding portion, and the folding portion is configured to cover at least part of the housing of the host.

41. The breast pump of claim 40, wherein the host comprises a charging hole covered by the folding portion.

42. The breast pump of claim 40, wherein an entire peripheral wall of the housing is wrapped by the folding portion.

43. A breast pump system, **characterized by** comprising:
a breast pump shield configured for fitting with a breast;
a milk storage assembly communicated with the breast pump shield and configured to receive and store a milk;
a host comprising a housing, wherein an accommodating chamber is provided in the housing, and the accommodating chamber is provided with a negative pressure assembly configured to apply a negative pressure to the milk storage assembly or the breast pump shield;
a power supply system provided in the accommodating chamber and electrically connected to the negative pressure assembly; and
a remote control configured to communicate with the negative pressure assembly and remotely control the negative pressure assembly to operate and stop; wherein the remote control comprises a housing and a control circuit board; the housing of the remote control is provided with a cavity body, and the control circuit board is provided in the cavity body; the housing of the remote control is provided with a control panel; the control panel is electrically connected to the control circuit board, and the remote control is detachably provided on an outer wall of the housing of the host or an outer wall of the milk storage assembly.

44. The breast pump system of claim 43, wherein an accommodating groove is provided at a top of the housing, and the remote control is accommodated in the accommodating groove and is detachably connected to an inner wall of the accommodating groove.

45. The breast pump system of claim 43, further comprising:
a third magnetic portion and a fourth magnetic portion magnetically attracted to the third magnetic portion;
wherein the third magnetic portion and the fourth magnetic portion are respectively provided on the housing of the remote control and an outer wall of the housing of the remote control.

46. The breast pump system of claim 43, wherein the remote control is detachably provided on the housing of the host and connected to the power supply system to provide power;
a connection mode between the remote control and the power supply system is one of a metal contact connection, a wireless connection or a wired connection;
the remote control further comprises a display screen provided with the housing of the remote control and electrically connected to the control circuit board; and
the remote control further comprises a watch strap configured to connect to the housing of the remote control.

47. The breast pump system of claim 43, wherein the milk storage assembly further comprises a dust cover detachably connected to the flange of the breast pump shield, and the remote control is detachably provided on the dust cover; and
a convex plate is formed at a middle of the dust cover, and the remote control is clamped to the convex plate so that the remote control is connected to the dust cover.
